Europäisches Patentamt

European Patent Office

Office européen des brevets

(11) Publication number: **0 227 899**
**B1**

(12) **EUROPEAN PATENT SPECIFICATION**

(45) Date of publication of patent specification: **16.01.91**

(21) Application number: **86114199.2**

(22) Date of filing: **14.10.86**

(51) Int. Cl.⁵: **A 61 M 5/00,** A 61 M 5/14,
B 65 D 33/14

(54) **Plastic hanger.**

(30) Priority: **25.11.85 US 801547**

(43) Date of publication of application:
**08.07.87 Bulletin 87/28**

(45) Publication of the grant of the patent:
**16.01.91 Bulletin 91/03**

(84) Designated Contracting States:
**BE CH DE FR GB IT LI NL SE**

(56) References cited:
**US-A-3 854 689**
**US-A-4 027 842**

(73) Proprietor: **ABBOTT LABORATORIES**
**14th Street and Sheridan Road North St**
**North Chicago, Illinois 60064 (US)**

(72) Inventor: **Brose, Robert C.**
**37380 N. Greenbay Road**
**Waukegan Illinois 60087 (US)**

(74) Representative: **Modiano, Guido et al**
**MODIANO, JOSIF, PISANTY & STAUB Modiano**
**& Associati Via Meravigli, 16**
**I-20123 Milano (IT)**

Courier Press, Leamington Spa, England.

EP 0 227 899 B1

## Description

Technical Field

The present invention relates generally to hanger devices, and, more particularly, to hangers which are designed to be packaged with intravenous solution bags.

Background Art

Hanger devices having elongated vertical bodies with hooks at both ends thereof are well known in the art, and have been used extensively in conjunction with intravenous solution bags and the like. Certain disadvantages exist in the packaging of these hanger devices, however. For example, since it is desirable to package the hangers in small boxes, they must be folded prior to insertion into the boxes. This folding, whether it is accomplished by hand or by machine, adds additional time and cost to the packaging of the hangers. Furthermore, since each hanger has two hooks, the hangers tend to become entangled, thereby hindering the sorting and packaging process.

Summary of the Invention

It is a primary object of the present invention to provide an improved hanger which is resistant to entanglement during packaging.

It is another object of this invention to provide such an improved hanger which is prefolded, and therefore need not be folded prior to insertion into its packaging.

A further object of this invention is to provide a hanger which is inexpensive to fabricate and package.

A related object is to provide a hanger which may be fabricated in a pre-folded and tied condition.

Other objects and advantages of the invention will be apparent from the following detailed description.

In accordance with the present invention, there is provided a one-piece hanger for suspending an intravenous solution bag or the like as defined in claim 1.

The inventive hanger has an elongated body having substantially inflexible hook members positioned at opposite ends thereof and each facing in the same direction. The elongated body is substantially resistant to folding throughout its length except at its longtiudinal center where there is a foldable region. According to the invention, the body has a U-shaped configuration wherein said foldable central region constitutes the bend of the U so that the hook members are adjacent and face each other. Integrally formed breakable tie means are provided to maintain said body in its preformed U-shaped configuration, until the tie means is broken to permit the body member to be straightened for use.

Brief Description of the Drawings

Figure 1 is a side elevational view of a hanger device embodying the prior art;

Figure 2 is a side view of the prior art hanger in the folded condition;

Figure 3 is a side elevational view of a hanger embodying the present invention;

Figure 4 is a section taken generally along line 4—4 in Figure 3;

Figure 5 is a section taken generally along line 5—5 in Figure 3;

Figure 6 is a section taken generally along line 6—6 in Figure 3.

Description of the Preferred Embodiments

While the invention will be described in connection with certain preferred embodiments, it will be understood that it is not intended to limit the invention to these particular embodiments. On the contrary, it is intended to cover all alternatives, modifications, and equivalents included within the spirit and scope of the invention as defined by the appended claims.

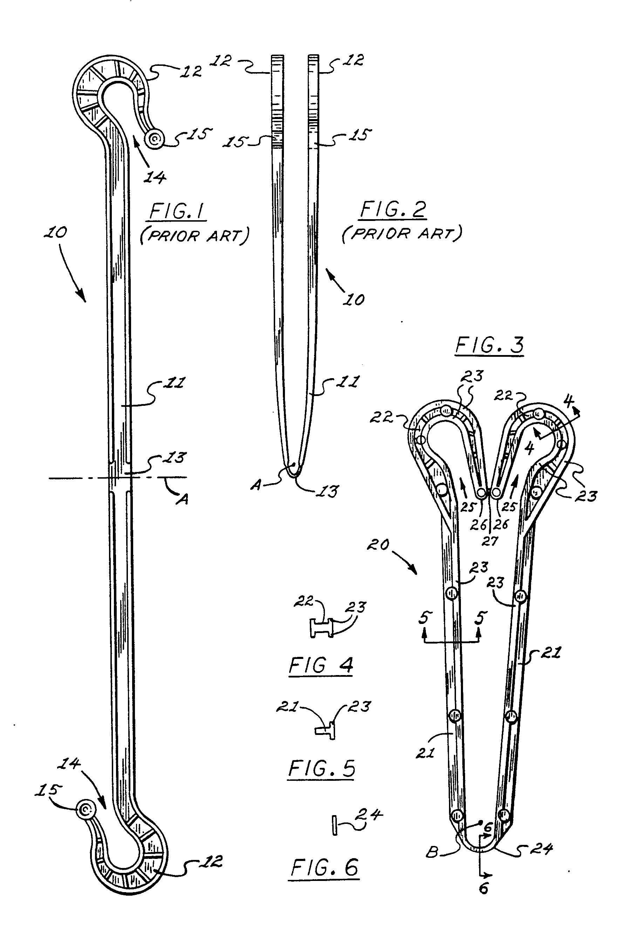

Turning now to the drawings and referring to Figure 1, there is shown a prior art hanger device 10 having an elongated body 11 with oppositely-facing hook members 12 at opposite ends thereof. The longitudinal center 13 of the known hanger device 10 has a substantially flat cross section such that this region is more flexible than the other regions. Since it is desirable to market the hanger devices 10 in small packages, each is folded at its longitudinally central region 13 prior to insertion into a package. The known device in the folded condition is shown in Figure 2. This folding, whether it is accomplished by hand or by machine, adds additional time and cost to the packaging of the hangers and is therefore undesirable. Since the prior art hanger devices 10 are molded such that the hook entrance gaps 14 and the hook member tips 15 are exposed, the hangers tend to become entangled during sorting and packaging, thereby increasing further the time and cost required for those processes. In addition, this tendency to entangle may make removal of the hangers from the packaging difficult.

In accordance with the present invention, there is provided a one-piece hanger comprising a foldable elongated body having substantially inflexible hook members positioned at opposite ends thereof, the hook members being substantially semi-circular in shape and defining hook entrance gaps, the body being formed in a U-shaped configuration so that the opposing hook members lie adjacent to each other with their defined hook entrance gaps facing the bend of the U; and integrally formed tie means for maintaining the body in the U-shaped configuration, the tie means being breakable so that the body member can be straightened at the foldable central region when use of the hanger is desired. Thus, in the illustrative embodiment of the invention shown in Figure 3, the inventive one-piece hanger 20 comprises a foldable elongated body 21 having hook members 22 at opposite ends thereof. The inventive hanger preferably comprises either flexible synthetic resins or flexible plastics, and is formed either by molding or by stamping.

In the preferred embodiment of the present invention, the foldable body 21 is substantially resistant to folding throughout its length, except at its longitudinal center where there is a foldable region. In addition, the hook members 22 are preferably formed such that they are substantially inflexible. As shown in the embodiment of Figure 3, the hook members 22 have strengthening flanges 23 integral with, and extending along, their peripheries such that they have I-shaped cross sections (Figure 4) and are substantially rigid. In comparison, the elongated body 21 has a T-shaped cross section throughout its length (see Figures 3 and 5), except at its longitudinally central region 24 which has a substantially flat cross section (see Figures 3 and 6). Due to its substantially flat cross section, the longitudinally central region 24 is quite pliable, and is therefore susceptible to folding and unfolding, whereas the other regions of the elongated body 21 are less pliable and generally resist folding.

In accordance with one important aspect of the present invention, the elongated body 21 is initially formed in a U-shaped configuration wherein the foldable longitudinally central region 24 is folded to constitute the bend of the U so that the opposing hook members 22 lie adjacent to each other. Because the inventive hanger 20 is initially formed in this U-shaped configuration, there is no need for additional folding prior to its insertion into a small package, and therefore, the packaging process is less time consuming and less costly.

Each of the hook members 22 defines a hook entrance gap 25 by which the hook member 22 loops over either an object from which the hanger 20 is to be suspended or an object which is to be suspended on the hanger. Unlike the prior art hanger device 10 of Figure 1, the inventive hanger 20 is formed so that both of the hook entrance gaps 25 of its hook members 22 face the bend of the U defined by the folded elongated body 21 (see Figure 3). As such, the tips 26 of the hook members 22 lie directly adjacent to one another when the hanger 20 is in its formed U-shaped configuration.

In accordance with another important aspect of the present invention, the hanger further comprises integrally formed tie means for maintaining the elongated body in its U-shaped configuration. More specifically, the tie means is formed integrally with the hanger and interconnects opposing members of the hanger (i.e., opposing body members, hook members or hook member tips), thereby maintaining the elongated body in its U-shaped configuration with the hook member tips lying directly adjacent to one another so that the hook members are not susceptible to becoming entangled with other objects.

As shown in the preferred emboidment of Figure 3, the tie means 27 comprises a short bridge of the plastic or synthetic resin from which the hanger 20 is formed and is integrally formed with the tips 26 of the hook members 22, thereby connecting the hook members 22 to one another and converting the hanger 20 into a continuous loop. Due to this conversion of the hanger 20 into a continuous loop, with the hook entrance gaps 25 lying on the inside of the loop, the hook member tips 26 are inwardly disposed. As a result, idential hangers 20 are less susceptible to becoming entangled with one another, resulting in less time consuming, and less costly, sorting and packaging processes.

The time means 27 is preferably formed such that it is resistant to accidental breakage (i.e., breakage due to jostling or vibration), and yet is easily breakable by a person wishing to utilize the hanger 20. In one embodiment which accommodates these opposing considerations, the tie means 27 comprises an integral short bridge interconnecting the hook member tips 26, and the tie means is formed from the flexible plastic or synthetic resin from which the hanger 20 is formed and has a diameter of approximately 0.025 cm (0.01 inches).

When use of the hanger 20 is desired, the tie means 27 is broken and the elongated body 21 is straightened (i.e., the foldable central region 24 is unfolded). The hanger 20 then has an appearance similar to the prior art hanger device shown in Figure 1, and is ready for use.

Significant advantages of the present invention over the known deice may be appreciated by reference to their respective folding axes. It will be noted that the known hanger device 10 folds about an axis A which, in Figure 1, is parallel with the plane of the drawings sheet. The folded prior art hanger is shown in Figure 2. By contrast, the inventive hanger 20 folds about its axis B which, in Figure 3, is perpendicular to the drawing sheet. The significance of this difference is that the present invention is susceptible to fabrication in the pre-folded, tied condition, either by molding or by stamping. Those skilled in the art will recognize that the prior art design does not lend itself to such simple fabrication. Rather, the hook member tips 15 of the prior art hanger device 10 can be connected to one another so as to alleviate the entanglement problem only by means of some post-fabrication process.

As can be seen from the foregoing detailed description, this invention provides an improved hanger which is inexpensive to manufacture, and which is inexpensive to package due to its resistance to entangelement and due to its formation in a pre-folded configuration.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. One-piece hanger (20) for suspending an intravenous solution bag of the type having a foldable elongated body (21) with substantially flexible hook members (22) positioned at opposite ends thereof, which hook members (22) are substantially semi-circular in shape and define hook entrance gaps (25), said one piece hanger (20) being characterized in that said foldable body (21) has a U-shaped configuration so that said opposing hook members lie adjacent to each other with their defined hook entrance gaps (25) facing the bend of the U, and tie means (27) integral with said hook members (22) for connecting said opposing hook members (22) to one another thereby maintaining said body (21) in said U-shaped configuration, said tie means (27) being breakable so that said opposing hook members (22) can be disconnected and said foldable body (21) can be straightened when the use of said hanger (20) is desired.

2. A hanger according to claim 1, characterized in that said foldable body (21) is substantially resistant to folding throughout its length except at its longitudinal center where there is a foldable region.

3. A hanger (20) according to claim 1 or 2, characterized in that it is comprised of flexible synthetic resins.

4. A hanger (20) according to claim 1 or 2, characterized in that it is comprised of flexible plastics.

## Patentansprüche

1. Einstückiger Aufhänger (20) zur Aufhängung eines Beutels für intravenöse Lösungen von dem Typ mit einem faltbar verlängerten Körper (21) mit im wesentlichen nicht biegsamen Hakenelementen (22) an seinen gegenüberliegenden Enden, welche Hakenenelemente (22) im wesentlichen halbkreisförmig in der Form sind und Hakenzugangsöffnungen (25) definieren, wobei der einstückige Aufhänger (20) dadurch gekennzeichnet ist, daß der faltbare Körper (21) eine U-förmige Konfiguration aufweist, so daß die gegenüberliegenden Hakenelemente mit ihrem definierten Hakenzugangsöffnungen (25) die der Biegung des U gegenüberliegen, aneinandergrenzend zueinander angeordnet sind, sowie mit den Hakenelementen (22) integral ausgebildete Bindeelemente (27), um die einander gegenüberliegenden Hakenelemente (22) miteinander zu verbinden und dadurch den Körper (21) in der U-förmigen Konfiguration zu halten, wobei die Bindeelemente (27) zerbrechbar sind, so daß die einander gegenüberliegenden Hakenelemente (22) getrennt werden können und der faltbare Körper (21) gerade ausgerichtet werden kann, wenn die Benutzung des Aufhängers (20) erwünscht ist.

2. Aufhänger nach Anspruch 1, dadurch gekennzeichnet, daß der faltbare Körper (21) über seine Länge im wesentlichen biegebeständig ist, außer an seiner auf die Länge bezongenen Mitte, so es eine biegbare Region gibt.

3. Aufhänger (20) nach Anspruch 1 oder 2, dadurch gekennzeichnet, daß er aus flexiblen synthetischen Kunststoffen besteht.

4. Aufhänger (20) nach Anspruch 1 oder 2, dadurch gekennzeichnet, daß er aus flexiblem Plastikmaterial besteht.

## Revendications

1. Organe de suspension monobloc (20) destiné à la suspension d'une poche de solution intraveineuse du type possédant un corps (21) allongé pliable et des éléments formant crochets (22) essentiellement rigides positionnés à ses extrémités opposées, lesquels éléments formant crochets (22) sont de forme essentiellement semi-circulaire et définissent des espaces (25) à l'entrée des corchets, ledit organe de suspension monobloc (20) étant caractérisé en ce que ledit corps pliable (21) possède une configuration en U de sorte que lesdits éléments formant crochets opposés sont mutuellement adjacents les espaces (25) définis à leur entrée faisant face à la courbure du U et le dispositif d'attache (27) intégré auxdits éléments formant crochets (22) pour relier lesdits éléments formant crochets opposés (22) entre euxmêmes maintenant ainsi ledit corps (21) dans ladite configuration en U ledit dispositif d'attache (27) étant cassable de sorte que lesdits éléments formant crochets opposés (22) peuvent être séparés et que ledit corps pliable (21) peut être redressé lorsqu'on désire utiliser ledit organe de suspension (20).

2. Organe de suspension selon la revendication 1, caractérisé en ce que ledit corps pliable (21) résiste essentiellement au pliage sur toute sa longueur, à l'exception de son centre longitudinal où se trouve une zone pliable.

3. Organe de suspension (20) selon la revendication 1 ou 2, caractérisé en ce qu'il est en résines synthétiques souples.

4. Organe de suspension (20) selon la revendication 1 ou 2, caractérisé en ce qu'il est en matières plastiques souples.

FIG.1
(PRIOR ART)

FIG.2
(PRIOR ART)

FIG. 3

FIG 4

FIG.5

FIG. 6

1